# EUROPEAN PATENT SPECIFICATION

(11) **EP 3 239 149 B1**
(45) Date of publication and mention of the grant of the patent: **27.01.2021**
(21) Application number: 15871510.2
(22) Date of filing: 27.03.2015
(51) Int. Cl.: C07D 409/10, A61P 3/10, A61P 27/02, A61P 25/00

(54) **CRYSTAL FORM I OF CANAGLIFLOZIN AND PREPARATION METHOD THEREOF**
KRISTALLINE FORM VON CANAGLIFLOZIN UND HERSTELLUNGSVERFAHREN DAFÜR
FORME CRISTALLINE I DE LA CANAGLIFLOZINE ET PROCÉDÉ DE PRÉPARATION DE CELLE-CI

(30) Priority: 25.12.2014 CN 201410820560
(43) Date of publication of application: 01.11.2017
(73) Proprietor: Chongqing Pharmaceutical Industrial Research Institute Co. Ltd., Chongqing 400061 (CN)
(72) Inventor: WANG, Fei, Chongqing 400061 (CN); ZHANG, Jian, Chongqing 400061 (CN); LIN, Meng, Chongqing 400061 (CN); TANG, Yuanfu, Chongqing 400061 (CN); CHEN, Hao, Chongqing 400061 (CN); LEI, Huangshu, Chongqing 400061 (CN)
(74) Representative: HGF
(86) International application number: PCT/CN2015/075180
(87) International publication number: WO 2016/101432

(56) References cited:
- WO-A1-2014/180872
- CN-A- 101 573 368
- CN-A- 101 801 371
- CN-A- 103 554 092
- CN-A- 103 588 762
- CN-A- 103 641 822
- CN-A- 103 896 930
- CN-A- 103 936 725
- CN-A- 103 980 262

## Description

### FIELD

The present invention belongs to the field of medicinal chemistry, and more particularly relates to a novel crystal form of Canagliflozin which is crystal form I of Canagliflozin, and a preparation method thereof.

### BACKGROUND

Canagliflozin, which has a chemical name of 1-(β-D-glucopyranosyl)-4-methyl-3-[5-(4-fluorophenyl)-2-thienylmethyl]benzene and a structure as shown in formula I, and can be prepared by the method disclosed in CN101801371.

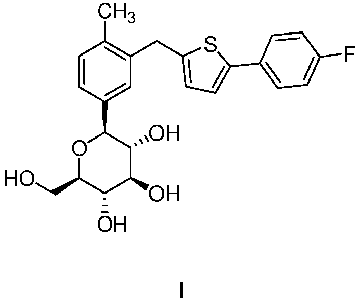

Canagliflozin is a selective type II sodium-glucose cotransporter protein (SGLT2) inhibitor which was developed by US Johnson & Johnson Company. On March 29, 2013, US Food and Drug Administration (FDA) approved that it can be used in combination with diet control and exercise so as to improve glycemic control in adults with type-2 diabetes mellitus.

Canagliflozin is a poorly water soluble compound which is generally used in a solid form in formulations, and therefore, researches on its crystal form are of great significance.

CN101573368 discloses a crystal form of a hemihydrate of Canagliflozin having characteristic diffraction peaks at 2θ values of 4.36°, 13.54°, 16.00°, 19.32° and 20.80° in its X-ray powder diffraction pattern using a CuK_{α} source, which crystal form is obtained by curing in ethyl acetate/diethyl ether/water or acetone/water system.

CN101801371 discloses another crystal form having characteristic diffraction peaks at 2θ values of 10.9°, 15.5°, 17.3°, 18.8° and 20.3° in its X-ray powder diffraction pattern using a CuK_{α} source, which crystal form is obtained by curing in ethyl acetate/n-heptane/water system.

WO2013064909 discloses co-crystals of Canagliflozin with L-proline, D-proline, L-phenylalanine, as well as an amorphous form of Canagliflozin.

CN103554092 discloses a crystal form B of Canagliflozin having characteristic diffraction peaks at 2θ values of 6.3°, 9.4° and 12.6° in its X-ray powder diffraction pattern using a CuK_{α} source, which crystal form is obtained by dissolving Canagliflozin in a mixed solvent of water and an organic solvent followed by slow evaporation at room temperature.

CN103588762 discloses a crystal form C of Canagliflozin having characteristic diffraction peaks at 2θ values of 6.5°, 9.8° and 16.4° in its X-ray powder diffraction pattern using a CuK_{α} source, which crystal form is obtained by dissolving Canagliflozin in a mixed solvent of water and an organic solvent followed by slow evaporation at room temperature. This patent further discloses a crystal form D of Canagliflozin having characteristic diffraction peaks at 2θ values of 6.8°, 13.6° and 20.5° in its X-ray powder diffraction pattern using a CuK_{α} source, which crystal form is obtained by heating the prepared crystal form C to 50∼90 °C.

CN103641822 discloses another crystal form of a hemihydrate of Canagliflozin having characteristic diffraction peaks at 2θ values of 3.86°, 15.46°, 17.30°, 18.80°, 19.10° and 20.26° in its X-ray powder diffraction pattern using a CuK_{α} source, which crystal form is obtained by dissolving Canagliflozin in a good solvent, and then adding a mixed solvent of a poor solvent and water to precipitate it.

CN103980261 discloses a crystal form A of Canagliflozin having characteristic diffraction peaks at 2θ values of 3.7°, 7.7°, 7.9°, 11.5°, 13.1°, 13.5°, 14.3°, 15.5°, 17.3°, 18.8°, 19.3°, 20.3°, 22.5°, 22.7°, 23.2° and 23.4° in its X-ray powder diffraction pattern using a CuK_{α} source, which crystal form is obtained by dissolving Canagliflozin in an alcoholic solvent to formulate a suspension at 0.05∼0.5 g/ml, dissolving the suspension at 15∼43°C and then adding 3∼10 times a solvating-out agent to precipitate it.

CN103980262 discloses another crystal form B of Canagliflozin having characteristic diffraction peaks at 2θ values of 3.4°, 6.6°, 12.6°, 13.2°, 15.3°, 15.6°, 16.5°, 19.4°, 19.8° and 23.7° in its X-ray powder diffraction pattern using a CuK_{α} source, which crystal form is obtained by dissolving Canagliflozin in an alcoholic solvent to formulate a solution at 0.1∼0.5 g/ml, and evaporating the solvent at 48∼70°C.

CN103936725 discloses another crystal form C of Canagliflozin having characteristic diffraction peaks at 2θ values of 3.4°, 6.5°, 12.7°, 15.8°, 19.8°, 24.3°, 24.8° and 29.1° in its X-ray powder diffraction pattern using a CuK_{α} source, which crystal form is obtained by dissolving Canagliflozin in an organic good solvent to formulate a solution at 0.05∼0.3 g/ml, adding a poor solvent after being dissolved, and precipitating it at -20∼10°C.

WO2014180872 discloses another crystal form of Canagliflozin having characteristic diffraction peaks at 2θ values of 5.4°, 6.7°, 13.2°, 16.1°, 19.6° and 24.1° in an X-ray powder diffraction pattern using a CuK_{α} source, which crystal form is obtained by converting an amorphous form of Canagliflozin in water.

The inventors prepared a novel crystal form of Canagliflozin during studying the crystal forms of Canagliflozin, which is prepared in a simple way, has stable physical and chemical properties, is easy to be stored and is suitable for being prepared into various preparations.

### SUMMARY

It is an object of the present invention to provide a novel crystal form of Canagliflozin, which has a simple preparation process, excellent physical and chemical stability and is suitable for manufacturing and industrial production of various preparations.

The novel crystal form of Canagliflozin provided in the present invention is defined herein as crystal form I of Canagliflozin.

The crystal form I of Canagliflozin of the present invention has characteristic diffraction peaks at positions with 2θ values of 4.4±0.2°, 8.4±0.2°, 16.8±0.2°, 17.5±0.2°, 18.0±0.2° and 22.8±0.2° in the X-ray powder diffraction pattern thereof.

The crystal form I of Canagliflozin of the present invention as described above further comprises characteristic diffraction peaks at positions with 2θ values of 12.1±0.2°, 12.6±0.2°, 15.3±0.2°, 19.3±0.2°, 20.4±0.2°, 22.2±0.2°, 23.0±0.2°, 24.6±0.2° and 26.6±0.2° in the X-ray powder diffraction pattern thereof.

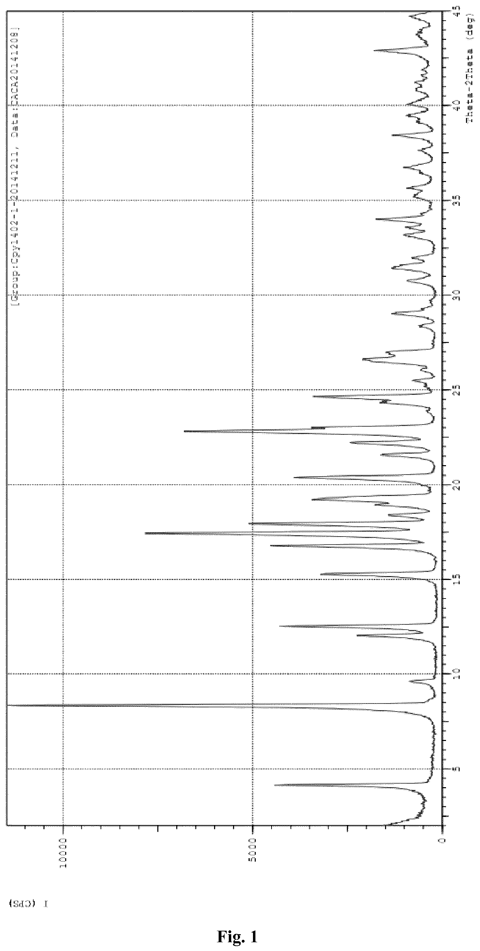

In an embodiment, the crystal form I of Canagliflozin of the present invention has characteristic diffraction peaks as shown in Figure 1 in the X-ray powder diffraction pattern thereof.

The crystal form I of Canagliflozin of the present invention has an endothermic peak between 90°C and 95°C, particularly reaches a peak at about 93°C in a DSC scanning graph thereof; as well as has about 3.97% of weight loss when heated to 180°C in a TGA scanning graph thereof.

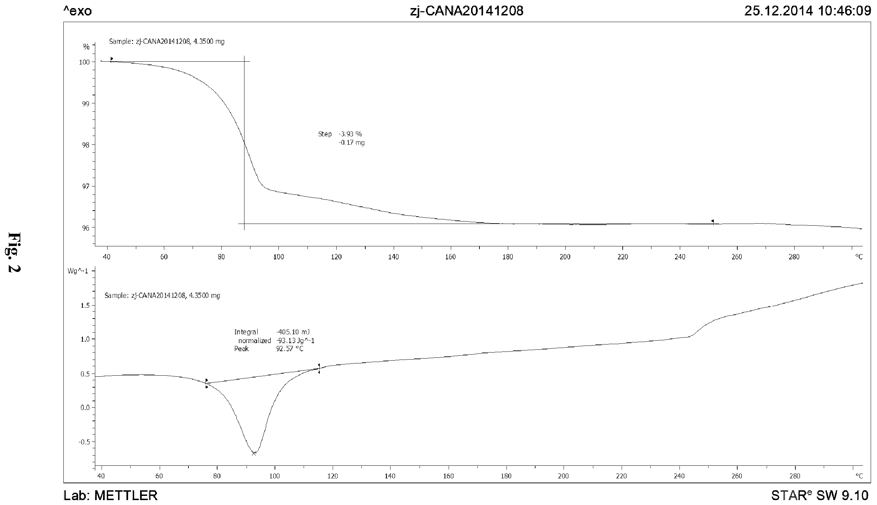

The crystal form I of Canagliflozin of the present invention has characteristics as shown in Figure 2 in the DSC-TGA scanning graph thereof.

The novel crystal form I of Canagliflozin of the present invention has a characteristic absorption peak at 1647 cm⁻¹ in the infrared absorption spectrum thereof.

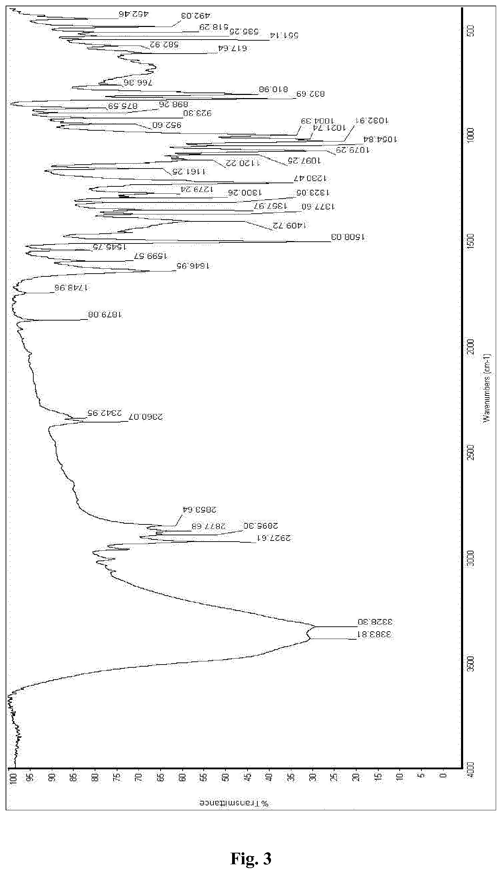

In an embodiment, the novel crystal form of Canagliflozin of the present invention has characteristics as shown in Figure 3 in the infrared absorption spectrum thereof.

The X-ray powder diffraction test on the crystal form I of Canagliflozin of the present invention was performed with a CuKα source (α=1.5406Å) from a Shimadzu XRD-6000 X-ray diffractometer, Japan, at ambient temperature and ambient humidity. During the test, due to a variety of factors such as particle size of the test sample, treatment method of the sample when testing, instrument, test parameters, test operations and so on, there will be some differences in the peak position or peak intensity of the measured X-ray powder diffraction patterns for the same crystal form. The experimental error of the 2θ values of the diffraction peaks in X-ray powder diffraction patterns may be within ± 0.2°. "Ambient temperature" is typically 0∼40°C and "ambient humidity" is typically 30%∼80% of relative humidity.

The DSC-TGA analysis of the crystal form I of Canagliflozin of the present invention was performed at ambient temperature and ambient humidity by using a Mettler 1100LF type instrument, Switzerland. The test was carried out by purging with a high-purity Ar gas at a flow rate of 50 ml/min and programmed warming at a rate of 10°C/min, with the range of temperature rise being from room temperature to 300°C. "Ambient temperature" is typically 0∼40°C and "ambient humidity" is typically 30%∼80% of relative humidity.

The IR spectrum analysis of the crystal form I of Canagliflozin of the present invention was tested by Fourier Transform Infrared Spectrometer (Nicolet Atavar FT-IR330) from Nicolet company at a relative humidity of typically less than 80% and a temperature of typically 15∼30°C. During the test, a tablet was pressed with KBr, and the spectrophotometer was calibrated with polystyrene (wavelength). During the test, due to a variety of factors (such as the size of the ground particles, the degree of tabletting, and the relative humidity in the air and so on), there will be some differences in the peak position or peak intensity of the measured IR spectra. The experimental error of the characteristic absorption peak values in the IR spectra may be within ±2 cm⁻¹.

An object of the present invention is to further provide a method for preparing crystal form I of Canagliflozin, which comprises heating and dissolving Canagliflozin in a mixed solvent of a good solvent and water, and then adding water to precipitate Canagliflozin.

In a specific embodiment, the method for preparing crystal form I of Canagliflozin of the present invention comprises the following steps:
1) dissolving Canagliflozin with a mixed solvent of a suitable good solvent and water to obtain a Canagliflozin solution, wherein the dissolving temperature is 30∼100°C, preferably 50∼80°C;
2) then reducing the temperature of the Canagliflozin solution to 20∼60°C, preferably 30∼50°C, and adding water to precipitate Canagliflozin;
3) separating the precipitated solid by filtration or centrifugation;
4) optionally, drying the separated solid at a drying temperature of generally 30∼80°C, preferably 40∼50°C, wherein the drying can be drying under an ambient pressure, or drying under a reduced pressure with the vacuum degree being typically 300∼760 mmHg, preferably 650∼760 mmHg.

In the specific embodiment as described above, in the method of the present invention, the suitable good solvent in step 1) includes methanol, ethanol, isopropanol, acetone, tetrahydrofuran, N,N-dimethyl formamide, dimethyl sulfoxide, N,N-dimethyl acetamide, dioxane, or a mixture thereof, preferably methanol, ethanol, isopropanol or a mixture thereof.

In the specific embodiment as described above, in the method of the present invention, the volume ratio of the good solvent to water is 1:1∼3. The weight-to-volume ratio of Canagliflozin to the good solvent is 1:3∼8 g/ml.

In a preferred specific embodiment, the method for preparing crystal form I of Canagliflozin of the present invention comprises the following steps:
1) dissolving Canagliflozin with a mixed solvent of a suitable good solvent and water to obtain a Canagliflozin solution, wherein a dissolving temperature is 50∼80°C, and the suitable good solvent is selected from methanol, ethanol, and isopropanol;
2) then reducing the temperature of the Canagliflozin solution to 30∼50°C, and adding water to precipitate Canagliflozin;
3) separating the precipitated solid by filtration or centrifugation;
4) optionally, drying the separated solid under a reduced pressure, wherein the drying temperature is 30∼80°C, and the vacuum degree is 650∼760 mmHg,
wherein the volume ratio of the suitable good solvent to water is 1:1∼3.

In the preferred specific embodiments as described above, the weight-to-volume ratio of Canagliflozin to the good solvent is 1:3∼8 g/ml.

In the embodiments as described above, in the method of the present invention, the precipitation of Canagliflozin is generally completed under a stirring condition.

To illustrate the stability of the crystal form I of Canagliflozin of the present invention, the crystal form I of Canagliflozin prepared in Example 1 was selected for stability studies, and the results are shown in the table below.

**Table: Stability testing results of crystal form I of Canagliflozin**

| test conditions | HPLC change | | whether the appearance changed | whether there being apparent moisture absorption | crystal form |
|---|---|---|---|---|---|
| | before placed | after placed | | | |
| placed at a temperature of 25±2°C and a humidity of RH92.5% for 30 days | 99.89% | 99.89% | No change | No apparent moisture absorption | Crystal form I |
| Placed at a temperature of 40±2°C for 30 days | 99.89% | 99.88% | No change | No apparent moisture absorption | Crystal form I |
| placed at a temperature of 60±2°C for 30 days | 99.89% | 99.87% | No change | No apparent moisture absorption | Crystal form I |

As can be seen from the above table, the crystal form I of Canagliflozin of the present invention has a good stability and is favourable to be prepared into various preparations.

The crystal form I of Canagliflozin of the present invention has a simple preparation process, which can be completed by using common equipments and mild conditions, and is suitable for industrialized production.

### BRIEF DESCRIPTION OF THE DRAWINGS

Figure 1 is an X-ray diffraction pattern of the crystal form I of Canagliflozin of the present invention.
Figure 2 is a DSC-TGA graph of the crystal form I of Canagliflozin of the present invention.
Figure 3 is an infrared spectrum of the crystal form I of Canagliflozin of the present invention.

### DETAILED DESCRIPTION OF EMBODIMENTS

Hereinafter, the present invention will be further illustrated in conjunction with examples, which allow those skilled in the art to understand the substance of the present invention more completely and are not intended to limit the scope of the invention in any way.

The X-ray powder diffraction patterns as described in the present invention were collected on a Shimadzu XRD-6000 X-ray diffractometer, Japan. The parameters of the X-ray powder diffraction analysis of the present invention were as follows:
X-ray reflection parameter: CuKα
CuKα source (α = 1.5406Å)
Voltage: 40 kilovolts (KV)
Current: 30 milliamperes (mA)
Divergence slit: automatic
Scanning mode: continuous
Scanning range: 2∼45 degrees
Sampling width: 0.02 degree
Scanning speed: 2 degree/minute

The DSC-TGA graphs of the crystal form I of Canagliflozin of the present invention were collected on a Mettler 1100LF type instrument, Switzerland. The parameters of the DSC-TGA analysis of the present invention were as follows:
Temperature range: room temperature∼300°C
Scanning rate: 10°C/min
Protection gas: Ar gas 50 ml/min

The IR spectra (KBr tablet) of the crystal form I of Canagliflozin of the present invention were collected on a Fourier Transform Infrared spectrometer (Nicolet Atavar FT-IR330) from US Nicolet company.

### Example 1

100 g Canagliflozin was dissolved in a mixed solvent of 300 ml methanol and 100 ml water at 50∼55°C, cooled to a temperature of 30∼35°C, and 200 ml water was added dropwise under stirring. After the completion of the addition, a mass of solid precipitated out and was filtered. The filter cake was dried under a reduced pressure at 700 ∼ 760 mmHg and 40 ∼ 50°C to obtain 96 g Canagliflozin, HPLC: 99.89%. The test result for X-ray powder diffraction is shown in Figure 1; the test result for DSC-TGA is shown in Figure 2; and the test result for infrared spectrum is shown in Figure 3.

### Example 2 Preparation of crystal form I of Canagliflozin

80 g Canagliflozin was dissolved in a mixed solvent of 350 ml ethanol and 150 ml water at 65∼70°C, cooled to a temperature of 35∼40°C, and 550 ml water was added dropwise under stirring. After the completion of the addition, a mass of solid precipitated out, cooled to room temperature, and filtered. The filter cake was dried under a reduced pressure at 650∼760 mmHg and 45∼50°C to obtain 67 g Canagliflozin, HPLC: 99.88%. It was confirmed by X-ray powder diffraction analysis as crystal form I of Canagliflozin.

### Example 3 Preparation of crystal form I of Canagliflozin

100 g Canagliflozin was dissolved in a mixed solvent of 800 ml isopropanol and 300 ml water at 70∼80°C, cooled to a temperature of 40∼50°C, and 2100 ml water was added dropwise under stirring. After the completion of the addition, a mass of solid precipitated out, cooled to room temperature and filtered. The filter cake was dried under a reduced pressure at 680∼760 mmHg and 45∼50°C to obtain 92 g Canagliflozin, HPLC: 99.92%. It was confirmed by X-ray powder diffraction analysis as a crystal form I of Canagliflozin.

## Claims

1. A crystal form I of Canagliflozin, having characteristic diffraction peaks at positions with 2θ values of 4.4±0.2°, 8.4±0.2°, 16.8±0.2°, 17.5±0.2°, 18.0±0.2°, and 22.8±0.2° in an X-ray powder diffraction pattern thereof.

2. The crystal form I of claim 1, further comprising characteristic diffraction peaks at positions with 2θ values of 12.1±0.2°, 12.6±0.2°, 15.3±0.2°, 19.3±0.2°, 20.4±0.2°, 22.2±0.2°, 23.0±0.2°, 24.6±0.2°, and 26.6±0.2° in the X-ray powder diffraction pattern thereof.

3. The crystal form I of claim 1 or 2, substantially having characteristic diffraction peaks as shown in Figure 1 in the X-ray powder diffraction pattern thereof.

4. The crystal form I of claim 1 or 2, having an endothermic peak between 90°C and 95°C in a DSC scanning graph thereof.

5. A method for preparing the crystal form I of Canagliflozin of claim 1, comprising heating and dissolving Canagliflozin in a mixed solvent of a suitable good solvent and water, then adding water to precipitate Canagliflozin.

6. The method of claim 5, specifically comprising the following steps:
1) dissolving Canagliflozin with a mixed solvent of a suitable good solvent and water to obtain a Canagliflozin solution, wherein the dissolving temperature is 30∼100°C;
2) reducing the temperature of the Canagliflozin solution to 20∼60°C, and adding water to precipitate Canagliflozin;
3) separating the precipitated solid by filtration or centrifugation;
4) optionally, drying the separated solid at atmospheric or reduced pressure conditions, wherein the drying temperature is 30∼80°C.

7. The method of claim 6, wherein the dissolving temperature in step 1) is 50∼80°C, the temperature in step 2) is 30∼50°C, and the drying temperature in step 4) is 40∼50°C.

8. The method of claim 5 or 6, wherein the suitable good solvent includes methanol, ethanol, isopropanol, acetone, tetrahydrofuran, N,N-dimethyl formamide, dimethyl sulfoxide, N,N-dimethyl acetamide, dioxane, or a mixture thereof.

9. The method of claim 8, wherein the suitable good solvent is methanol, ethanol, isopropanol or a mixture thereof.

10. The method of claim 5 or 6, wherein the volume ratio of the suitable good solvent to water is 1:1-3.

## Patentansprüche

1. Kristallform I von Canagliflozin, die charakteristische Beugungspeaks an Positionen mit 2θ-Werten von 4,4±0,2 °, 8,4±0,2 °, 16,8±0,2 °, 17,5±0,2 °, 18,0±0,2 ° und 22,8±0,2 ° in einem Röntgenpulverbeugungsmuster davon aufweist.

2. Kristallform I nach Anspruch 1, ferner umfassend charakteristische Beugungspeaks an Positionen mit 2θ-Werten von 12,1±0,2 °, 12,6±0,2 °, 15,3±0,2 °, 19,3±0,2 °, 20,4±0,2 °, 22,2±0,2 °, 23,0±0,2 °, 24,6±0,2 ° und 26,6±0,2 ° in dem Röntgenpulverbeugungsmuster davon.

3. Kristallform I nach Anspruch 1 oder 2, im Wesentlichen charakteristische Beugungspeaks wie in Figur 1 gezeigt in dem Röntgenpulverbeugungsmuster davon aufweisend.

4. Kristallform I nach Anspruch 1 oder 2, einen endothermen Peak zwischen 90 °C und 95 °C in einem DSC-Scangraphen davon aufweisend.

5. Verfahren zur Herstellung der Kristallform I von Canagliflozin nach Anspruch 1, umfassend das Erwärmen und Auflösen von Canagliflozin in einem gemischten Lösungsmittel aus einem geeigneten guten Lösungsmittel und Wasser, dann das Hinzufügen von Wasser, um Canagliflozin zu präzipitieren.

6. Verfahren nach Anspruch 5, insbesondere die folgenden Schritte umfassend:
1) Auflösen von Canagliflozin mit einem gemischten Lösungsmittel aus einem geeigneten guten Lösungsmittel und Wasser, um eine Canagliflozin-Lösung zu erhalten, wobei die Auflösungstemperatur 30∼100 °C beträgt;
2) Reduzieren der Temperatur der Canagliflozin-Lösung auf 20∼60 °C, und Hinzufügen von Wasser, um Canagliflozin zu präzipitieren;
3) Trennen des präzipitierten Feststoffes durch Filtration oder Zentrifugation;
4) optional Trocknen des getrennten Feststoffes bei atmosphärischen oder reduzierten Druckbedingungen, wobei die Trocknungstemperatur 30∼80 °C beträgt.

7. Verfahren nach Anspruch 6, wobei die Auflösungstemperatur in Schritt 1) 50∼80 °C beträgt, die Temperatur in Schritt 2) 30∼50 °C beträgt und die Trocknungstemperatur in Schritt 4) 40∼50 °C beträgt.

8. Verfahren nach Anspruch 5 oder 6, wobei das geeignete gute Lösungsmittel Methanol, Ethanol, Isopropanol, Aceton, Tetrahydrofuran, N,N-Dimethylformamid, Dimethylsulfoxid, N,N-Dimethylacetamid, Dioxan oder eine Mischung davon beinhaltet.

9. Verfahren nach Anspruch 8, wobei das geeignete gute Lösungsmittel Methanol, Ethanol, Isopropanol oder eine Mischung davon ist.

10. Verfahren nach Anspruch 5 oder 6, wobei das Volumenverhältnis des geeigneten guten Lösungsmittels zu Wasser 1:1-3 beträgt.

## Revendications

1. Forme cristalline I de la Canagliflozine, ayant des pics de diffraction caractéristiques à des positions avec des valeurs 2θ de 4,4 ± 0,2°, 8,4 ± 0,2°, 16,8 ± 0,2°, 17,5 ± 0,2°, 18,0 ± 0,2°, et 22,8 ± 0,2° dans un profil de diffraction des rayons X sur poudre de celle-ci.

2. Forme cristalline I selon la revendication 1, comprenant en outre des pics de diffraction caractéristiques à des positions avec des valeurs 2θ values de 12,1 ± 0,2°, 12,6 ± 0,2°, 15,3 ± 0,2°, 19,3 ± 0,2°, 20,4 ± 0,2°, 22,2 ± 0,2°, 23,0 ± 0,2°, 24,6 ± 0,2°, et 26,6 ± 0,2° dans le profil de diffraction des rayons X sur poudre de celle-ci.

3. Forme cristalline I selon la revendication 1 ou 2, ayant substantiellement des pics de diffraction caractéristiques comme est montré sur la Figure 1 dans le profil de diffraction des rayons X sur poudre de celle-ci.

4. Forme cristalline I selon la revendication 1 ou 2, ayant un pic endothermique compris entre 90 °C et 95 °C dans un graphique de balayage DSC de celle-ci.

5. Procédé destiné à préparer la forme cristalline I de la Canagliflozine selon la revendication 1, comprenant le chauffage et la dissolution de Canagliflozine dans un solvant mélangé d'un bon solvant adapté et d'eau, puis l'ajout d'eau pour faire précipiter la Canagliflozine.

6. Procédé selon la revendication 5, comprenant spécifiquement les étapes suivantes :
1) la dissolution de Canagliflozine avec un solvant mélangé d'un bon solvant adapté et d'eau pour obtenir une solution de Canagliflozine, dans lequel la température de dissolution est de 30∼100 °C ;
2) la réduction de la température de la solution de Canagliflozine jusqu'à 20∼60 °C, et l'ajout d'eau pour faire précipiter la Canagliflozine ;
3) la séparation du solide précipité par filtration ou centrifugation ;
4) facultativement, le séchage du solide séparé dans des conditions de pression atmosphérique ou réduite, dans lequel la température de séchage est de 30∼80 °C.

7. Procédé selon la revendication 6, dans lequel la température de dissolution dans l'étape 1) est de 50∼80 °C, la température dans l'étape 2) est de 30∼50 °C, et la température de séchage dans l'étape 4) est de 40∼50°C.

8. Procédé selon la revendication 5 ou 6, dans lequel le bon solvant adapté inclut le méthanol, l'éthanol, l'isopropanol, l'acétone, le tétrahydrofurane, le N,N-diméthylformamide, le diméthylsulfoxyde, le N,N-diméthylacétamide, le dioxane, ou un mélange de ceux-ci.

9. Procédé selon la revendication 8, dans lequel le bon solvant adapté est le méthanol, l'éthanol, l'isopropanol ou un mélange de ceux-ci.

10. Procédé selon la revendication 5 ou 6, dans lequel le rapport volumique du bon solvant adapté et de l'eau est 1/1 à 3.
